# EUROPEAN PATENT SPECIFICATION

(11) **EP 3 181 105 B1**
(45) Date of publication and mention of the grant of the patent: **13.06.2018**
(21) Application number: 16002643.1
(22) Date of filing: 13.12.2016
(51) Int. Cl.: A61F 13/20, A61F 13/34

(54) **PROCESS FOR MANUFACTURING A TAMPON WITH INTEGRATED THERAPEUTICAL MEANS**
VERFAHREN ZUR HERSTELLUNG EINES TAMPONS MIT INTEGRIERTEN THERAPEUTISCHEN MITTELN
PROCÉDÉ DE FABRICATION D'UN TAMPON INTÉGRANT UN MOYEN THÉRAPEUTIQUE

(30) Priority: 14.12.2015 SI 201500297
(43) Date of publication of application: 21.06.2017
(73) Proprietor: Medicalsanomed AG, 8852 Altendorf (CH)
(72) Inventor: BRDNIK, Tomaz, 1293 Smarje-Sap (SI)
(74) Representative: Borstar, Dusan

(56) References cited:
- EP-A1- 0 422 660
- EP-A1- 2 417 954
- US-A- 3 559 646
- US-A- 4 335 720
- US-A1- 2007 128 254
- US-A1- 2010 136 088

## Description

The present invention refers to a process of manufacturing of a tampon, namely either a digital or applicator tampon with integrated means for performing medical treatment in the area of vaginal cavity. In accordance with International Patent Classification, such inventions belong to human necessities, substantially to medical science and hygiene, namely to tampons, in particular to catamenial tampons. Such inventions according to IPC⁸ belong to the class A 61 F 13/20.

In this, the purpose of the invention is to create a process of manufacturing a tampon of the previously described art, which could be manufactured in mass production by means of existing technology and equipment presently known to those skilled in the art, and into which either a solid or liquid therapeutic means could be introduced during performing of commonly known steps in said method of manufacturing, wherein such obtained tampon would have to retain its commonly known appearance including all functioning features, which are known to persons skilled in the art are also commonly known and acceptable to persons using such hygienic tampons.

A hygienic tampon as such is disclosed e.g. in EP 0 422 660 and consists of a non-woven fibrous semi-product, which is obtain by winding of a non-woven fibrous band around a longitudinal axis, by which at least approximately cylindrical semi-product is produced, which is then by means of radial compression generated by appropriate jaws, e.g. by means of an apparatus according to DE 19 825 877, transformed into a tampon. Such obtained tampon in principle still has cylindrical shape, and is furnished with grooves on its external surface, which are pressed radially inwards and extend longitudinally, namely parallel to said longitudinal i.e. central axis of the tampon. Such tampons are known to those skilled in the art as "digital" tampons. Between each two neighboring grooves there is a rib, and each diameter of the tampon is determined by the outer circumference of said ribs, while an essentially compressed core of the tampon is determined by bottom surfaces of said grooves. A further relatively smooth compression in the area of said ribs leads to convexity and bulky shape of lateral surfaces of said ribs, so that the lateral surfaces of each neighboring ribs are placed in contact with each other, and a tubular space is formed between each neighboring ribs adjacent to said core; which extend parallel to said longitudinal axis. Such concept should lead to several benefits, among others to improvement of buckling strength, bending strength, and in particular to improvement of specific absorptiveness and absorption rate in the longitudinal direction of such tampon. Stability of a tampon is of crucial importance in view of reliability on the side of each particular user, and is in particular important during insertion of a tampon. In addition to said buckling strength, properly insertion also relies on smoothness of the external surface of the tampon, which is in contact with mucosa, as well as on friction coefficient, which should be as low as possible.

Moreover, a tampon is disclosed in EP 2 417 954 A1, which is on its proximal end portion furnished with a blind hole having diameter between 10 - 45%, and preferably 25 - 35% relative to diameter of the tampon, but said opening is manufactured exclusively for the purposes of improving absorptiveness of the tampon.

Still further, a tampon is disclosed in WO 2009/000220, into which a therapeutic means is integrated, namely a permanent magnet, which is presented in a drawing and appears to be arranged in the area of longitudinal axis of the tampon, wherein there is no hint or explanation at all, how such tampon can be manufactured. US4335720 discloses a catamenial tampon having a hollow core opening, said opening being used as a container for a medicament The tampon is manufactured by die compression.

US2007/0128254 discloses a method for manufacturing a medicated tampon assembly having a tampon body, the method comprising: positioning a dosage form in a holder; heating the dosage form; placing the dosage form and the tampon body in contact with each other; and allowing the dosage form to cool. The present invention refers to a process of manufacturing a tampon with integrated therapeutic means, comprising
- a first step, in which a band is produced, the width of which at least approximately corresponds to the length of said tampon and which consists of absorptive cotton and/or cellulose fibers, optionally with added synthetic fibers;
- a second step, in which said band is helically wound around a geometric axis, which extends transversally with respect to said band and at the same time represents a central longitudinal axis of said tampon, wherein a string is inserted among threads, the one end of which protrudes outwardly from the distal end portion of said tampon, by which a substantially cylindrical semi-product consisting of said spirally wound absorptive fibrous material is obtained;
- a third step, in which said semi-product consisting of said spirally wound absorptive fibrous material is radially compressed along its overall length in several directions thereof namely in six up to twelve directions extending equidistantly along the circumference thereof, by which ribs and grooves are formed, which are alternatively and substantially equidistantly arranged along the circumference thereof, and which extend either linearly or spirally between the proximal end portion and the distal end portion;
- a fourth step, in which a substantially hemispherical tip is formed on said tampon by means of longitudinal compression in a direction of said longitudinal axis thereof.

The invention proposes that between said third and fourth step, namely upon finishing said third step including radial compression due to forming said ribs and grooves, and prior to radial compression due to forming said tip of a tampon, the last is furnished with a blind hole, which extends coaxially with said central longitudinal axis of the tampon, upon which a therapeutic means is introduced into said hole, which is then followed by performing said fourth step, namely said compression in axial direction due to forming said tip of the tampon.

In this, each used therapeutic means can be solid, preferably a permanent magnet, and prior to introduction of said therapeutic means also an adhesive can be introduced into said blind hole. Alternatively, said therapeutic means can also be liquid.

Now the invention will be described in detail on the basis of an embodiment, which is presented in the enclosed drawings, wherein
- Figs. 1 - 9: schematically present a sequence of steps when performing a process according to the invention;
- Fig. 10: is a schematic presentation of an apparatus for manufacturing a tampon obtained according to the invention, in front view;
- Fig. 11: presents said apparatus according to Fig. 10 in cross-section along the plane A - A; and

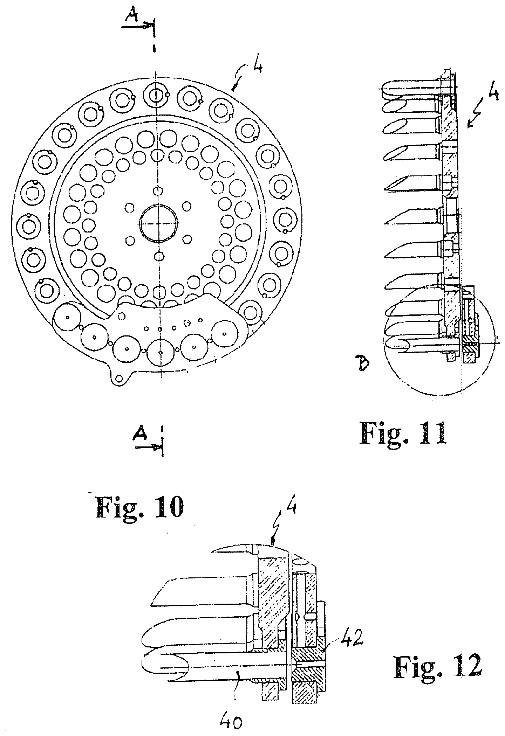

- Fig. 11: presents a detail B according to Fig. 11.

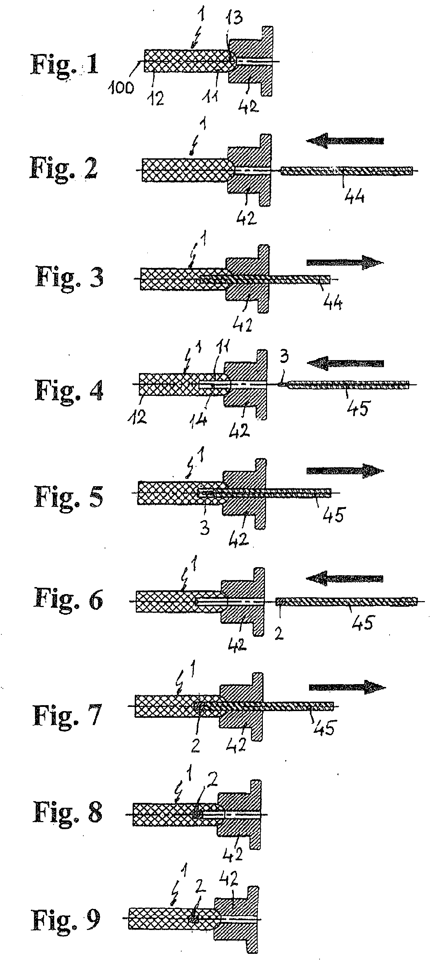

In Figs. 1 - 9 a sequence of single steps are schematically presented, which occur by performing a process according to the invention, namely those steps, which relate to preparing of a blind hole 14 including introduction of each therapeutic means 2 into a tampon 1, which is then followed by forming of a tip 13 on the proximal end portion 11 of the tampon 1.

Such process of manufacturing a tampon 1 with integrated therapeutic means can be performed by means of apparatus like that one, which is disclosed in EP 0 623 333 A2. Such apparatus comprises a rotational revolving tool 4, which is schematically presented in Figs. 1 - 12.

Consequently, during the first step, which is not separately shown in the drawings, a band is produced, the width of which at least approximately corresponds to the length of said tampon 1 and which consists of absorptive cotton and/or cellulose fibers, optionally with added synthetic fibers.

During the second step, which is also not separately shown in the drawings, said band is helically wound around a geometric axis, which extends transversally with respect to said band and at the same time represents a central longitudinal axis 100 of said tampon 1, wherein a string is inserted among threads, the one end or which protrudes outwardly from the distal end portion 12 of said tampon 1, by which a substantially cylindrical semi-product consisting of said spirally wound absorptive fibrous material is obtained.

During the third step, which is also not separately shown in the drawings, said semi-product consisting of said spirally wound absorptive fibrous material is radially compressed along its overall length in several directions thereof, namely in six up to twelve directions extending in equidistantly along the circumference thereof, by which ribs and grooves are formed, which are alternatively and substantially equidistantly arranged along the circumference thereof, and which extend either linearly or spirally between the proximal end portion 11 and the distal end portion 12.

The invention proposes that upon finishing said third step including radial compression due to forming said ribs and grooves, and prior to radial compression due to forming said tip 13 of the tampon 1, the last is furnished with a blind hole 14, which is located at its proximal end portion 11 and extends coaxially with said central longitudinal axis 100 of the tampon 1, upon which each therapeutic means 2 is introduced into said hole 14, which is presented in Figs. 6 and 7, upon which the fourth step is performed, which includes forming said tip 13 of the tampon 1, which is presented in Figs. 8 and 9.

To this aim, apertures 40 are furnished on said tool 4, into which the tampon 1 can be inserted, which has been a prior to that radially compressed and is therefore furnished with ribs and grooves, which extend between its proximal end portion 11 and distal end portion 12. Moreover, said tool comprises two guides 42, which are spaced apart from each other in the circumferential direction of said tool 4 and extend coaxially with each aperture 40 in the tool 4, and into which two pins 44, 45 can be inserted.

The first pin 44 is used for the purposes of forming said blind hole 14 in the proximal end portion 11 of the tampon, while the second pin 45 is used for the purposes of introducing said therapeutic means 2 into said hole 14 prior to forming the tip 13 of the tampon 1.

Said therapeutic means 2 can either be solid or liquid. When the therapeutic means 2 is solid some adhesive 3 can be introduced into said blind hole 14 prior to its introduction there-into, by which in particular in mass production of tampons 1 said therapeutic means 2 is retained within said blind hole 14 by removing the pin 45 therefrom. This is in particular useful when said therapeutic means 2 is a permanent magnet. However, when said therapeutic means 2 is liquid, then said pin 45 can be a tube and can operate similarly like a syringe.

After that, namely upon introduction of said therapeutic means 2, a further step is performed, in which a substantially hemispherical tip 13 is formed on said tampon 1 by means of longitudinal compression in a direction of said longitudinal axis 100 thereof, which is shown in Fig. 9, or, when desired, a correspondingly thickened hemi-spherical tip 13, which is commonly used by applicator tampons.

## Claims

1. Process of manufacturing a tampon (1) with integrated therapeutic means (2), comprising
- a first step, in which a band is produced, the width of which at least approximately corresponds to the length of said tampon (1) and which consists of absorptive cotton and/or cellulose fibers, optionally with added synthetic fibers;
- a second step, in which said band is helically wound around a geometric axis, which extends transversally with respect to said band and at the same time represents a central longitudinal axis (100) of said tampon (1), wherein a string is inserted among threads, the one end of which protrudes outwardly from the distal end portion (12) of said tampon (1), by which a substantially cylindrical semi-product consisting of said spirally wound absorptive fibrous material is obtained;
- a third step, in which said semi-product consisting of said spirally wound absorptive fibrous material is radially compressed along its overall length in several directions thereof, namely in six up to twelve directions extending equidistantly along the circumference thereof, by which ribs and grooves are formed, which are alternatively and substantially equidistantly arranged along the circumference thereof, and which extend either linearly or spirally between the proximal end portion (11) and the distal end portion (12);
- a fourth step, in which a substantially hemispherical tip (13) is formed on said tampon (1) by means of longitudinal compression in a direction of said longitudinal axis (100) thereof, **characterized in that**
between said third and fourth step, namely upon finishing said third step including radial compression due to forming said ribs and grooves, and prior to axial compression due to forming said tip (13) of a tampon (1), the last is furnished with a blind hole (14), which extends coaxially with said central longitudinal axis (100) of the tampon (1), upon which a therapeutic means (2) is introduced into said hole (14), which is then followed by performing said fourth step, namely said compression in axial direction (100) due to forming said tip (13) of the tampon (1).

2. Process according to Claim 1, **characterized in that** cach used therapeutic means (2) is solid.

3. Process according to Claim 2, **characterized in that** prior to introduction of said therapeutic means (2) also an adhesive (3) is introduced into said blind hole (14).

4. Process according to Claim 1, **characterized in that** said therapeutic means (2) is a permanent magnet.

5. Process according to Claim 1, **characterized in that** each used therapeutic means (2) is liquid.

## Patentansprüche

1. Verfahren zur Herstellung eines Tampons (1) mit integrierten therapeutischen Mitteln (2), umfassend
- einen ersten Schritt, in dem ein Band hergestellt wird, dessen Breite zumindest in etwa der Länge des Tampons (1) entspricht und welches aus absorbierenden Baumwolle- und/oder Cellulosefasern, gegebenenfalls mit hinzugefügten Chemiefasern, besteht;
- einen zweiten Schritt, in dem das Band spiralförmig um eine geometrische Achse gewickelt wird, die sich quer zum Band erstreckt und dabei eine Mittellängsachse (100) des Tampons (1) bildet, wobei eine Schnur zwischen Fäden eingefügt wird, deren eines Ende von dem distalen Endabschnitt (12) des Tampons (1) nach außen vorsteht, wodurch ein im Wesentlichen zylindrisches halbfertiges Produkt, das aus dem spiralförmig aufgewickelten, absorbierenden faserigen Material besteht, erhalten wird;
- einen dritten Schritt, in dem das halbfertige Produkt, das aus dem spiralförmig aufgewickelten, absorbierenden faserigen Material besteht, über seine gesamte Länge in mehrere Richtungen von diesem radial komprimiert wird, nämlich in sechs bis zwölf Richtungen, die sich in gleichen Abständen über seinen Umfang erstrecken, wodurch Rippen und Nuten gebildet werden, die abwechselnd und im Wesentlichen in gleichen Abständen über seinen Umfang angeordnet sind und sich entweder linear oder spiralförmig zwischen dem proximalen Endabschnitt (11) und dem distalen Endabschnitt (12) erstrecken;
- einen vierten Schritt, in dem eine im Wesentlichen halbkugelförmige Spitze (13) auf dem Tampon (1) durch eine Längskomprimierung in eine Richtung der Längsachse (100) von diesem gebildet wird, **dadurch gekennzeichnet, dass**
- zwischen dem dritten und dem vierten Schritt, nämlich nach dem Beenden des dritten Schritts einschließlich der radialen Komprimierung auf Grund der Bildung der Rippen und Nuten und vor der axialen Komprimierung auf Grund der Bildung der Spitze (13) eines Tampons (1), dieser mit einem Sackloch (14) versehen wird, das sich koaxial mit der Mittellängsachse (100) des Tampons (1) erstreckt, woraufhin ein therapeutisches Mittel (2) in das Loch (14) eingefügt wird, dann gefolgt vom Ausführen des vierten Schritts, nämlich der Komprimierung in axialer Richtung (100) auf Grund der Bildung der Spitze (13) des Tampons (1).

2. Verfahren gemäß Anspruch 1, **dadurch gekennzeichnet, dass** jedes verwendete therapeutische Mittel (2) fest ist.

3. Verfahren gemäß Anspruch 2, **dadurch gekennzeichnet, dass** vor der Einbringung der therapeutischen Mittel (2) auch ein Klebstoff (3) in das Sackloch (14) eingebracht wird.

4. Verfahren gemäß Anspruch 1, **dadurch gekennzeichnet, dass** das therapeutische Mittel (2) ein Dauermagnet ist.

5. Verfahren gemäß Anspruch 1, **dadurch gekennzeichnet, dass** jedes verwendete therapeutische Mittel (2) flüssig ist.

## Revendications

1. Procédé de fabrication d'un tampon (1) avec un moyen thérapeutique (2) intégré, comprenant
- une première étape, dans laquelle une bande est produite, dont la largeur correspond au moins approximativement à la longueur dudit tampon (1) et qui consiste en des fibres de coton et/ou de cellulose absorbantes, facultativement avec des fibres synthétiques ajoutées ;
- une deuxième étape, dans laquelle ladite bande est enroulée hélicoïdalement autour d'un axe géométrique, qui s'étend transversalement par rapport à ladite bande et en même temps représente un axe longitudinal central (100) dudit tampon (1), dans lequel une corde est insérée entre les fils, dont une extrémité fait saillie vers l'extérieur depuis la partie d'extrémité distale (12) dudit tampon (1), ce par quoi un demi-produit sensiblement cylindrique consistant en ladite matière fibreuse absorbante enroulée en spirale est obtenu ;
- une troisième étape, dans laquelle ledit demi-produit consistant en ladite matière fibreuse absorbante enroulée en spirale est compressé radialement sur la totalité de sa longueur dans plusieurs sens de celui-ci, nommément dans six à douze sens s'étendant de manière équidistante le long de la circonférence de celui-ci, ce par quoi des nervures et des rainures sont formées, qui sont agencées de manière alternée et sensiblement équidistante le long de la circonférence de celui-ci, et qui s'étendent de manière linéaire ou en spirale entre la partie d'extrémité proximale (11) et la partie d'extrémité distale (12) ;
- une quatrième étape, dans laquelle un bout sensiblement hémisphérique (13) est formé sur ledit tampon (1) au moyen d'une compression longitudinale dans un sens dudit axe longitudinal (100) de celui-ci, **caractérisé en ce que** entre lesdites troisième et quatrième étapes, nommément à la fin de ladite troisième étape incluant une compression radiale due à la formation desdites nervures et rainures, et avant une compression axiale due à la formation dudit bout (13) d'un tampon (1), ce dernier est doté d'un trou borgne (14), qui s'étend coaxialement avec ledit axe longitudinal central (100) du tampon (1), ce par quoi un moyen thérapeutique (2) est introduit dans ledit trou (14), ce qui est ensuite suivi par l'exécution de ladite quatrième étape, nommément ladite compression dans un sens axial (100) due à la formation dudit bout (13) du tampon (1).

2. Procédé selon la revendication 1, **caractérisé en ce que** chaque moyen thérapeutique (2) utilisé est solide.

3. Procédé selon la revendication 2, **caractérisé en ce que**, avant l'introduction dudit moyen thérapeutique (2), également un adhésif (3) est introduit dans ledit trou borgne (14).

4. Procédé selon la revendication 1, **caractérisé en ce que** ledit moyen thérapeutique (2) est un aimant permanent.

5. Procédé selon la revendication 1, **caractérisé en ce que** chaque moyen thérapeutique (2) utilisé est liquide.
